# EUROPEAN PATENT APPLICATION

(11) **EP 4 796 100 A1**
(43) Date of publication of application: **26.08.2026**
(21) Application number: 25890398.8
(22) Date of filing: 22.01.2025
(51) Int. Cl.: A61K 8/02, A61K 8/19, A61Q 1/00, A61Q 1/02, A61Q 1/12

(54) **PREPARATION METHOD FOR POWDERY COSMETIC PRODUCT HAVING COLOR GRADIENT EFFECT, AND POWDERY COSMETIC PRODUCT**

(30) Priority: 02.12.2024 CN 202411763215
(71) Applicant: A & H International Cosmetics Co., Ltd, Shanghai 201415 (CN)
(72) Inventor: TIAN, Yong, Shanghai 201415 (CN); CHEN, Jinhai, Shanghai 201415 (CN); ZHANG, Bin, Shanghai 201415 (CN); LIU, Siyu, Shanghai 201415 (CN); WU, Yidi, Shanghai 201415 (CN); SHEN, Jie, Shanghai 201415 (CN); SHIN, Bokchul, Shanghai 201415 (CN); ZHANG, Jinnv, Shanghai 201415 (CN); TIAN, Yuncai, Shanghai 201415 (CN)
(74) Representative: Valea AB
(86) International application number: PCT/CN2025/074063
(87) International publication number: WO 2026/118170

(57) **Abstract**

The present application provides a method for preparing a powder cosmetic with a gradient color effect and a powder cosmetic. The method comprises the following steps: mixing; color laydown: providing a mold, the mold having at least one cavity with an upward opening; taking at least one water-soluble pigment, diluting them separately to obtain at least one pigment solution, and dot-coating the at least one pigment solution onto at least one preset position on the bottom of the cavity; filling; freezing: freezing the material system within the mold at a low temperature to obtain a powder masterbatch; demolding: separating the powder masterbatch from the mold; drying: drying the demolded powder masterbatch to obtain a powder cosmetic with a gradient color effect; wherein the concentration of the pigment solution is from 0.1 % to 10% by mass; and the viscosity of the mixed material system at 25°C is from 1000 to 15000 cP. The cosmetic powder prepared by the preparation method of the present application can achieve natural blending and gradient color effects.

## Description

### CROSS REFERENCE TO RELATED APPLICATION

The present application claims priority to Chinese Patent Application No. 202411763215.9, entitled "METHOD FOR PREPARING POWDER COSMETIC WITH GRADIENT COLOR EFFECT AND POWDER COSMETIC" filed on December 2, 2024, the entire contents of which are incorporated herein by reference.

### TECHNICAL FIELD

The present application belongs to the technical field of daily cosmetics, and specifically relates to a method for preparing a powder cosmetic with a gradient color effect and a powder cosmetic.

### BACKGROUND

Powder cosmetics can modify facial features and increase the luster of makeup. For example, highlighter powder can locally brighten and enhance facial contours, making base makeup more radiant, combating dullness, and creating a visual contrast with light and shadow, thereby increasing the three-dimensionality of facial features.

Existing multi-color powder cosmetics on the market are mostly pressed powders. Their manufacturing process is complex, production efficiency is low, and the surface color is either irregular or has an obvious patchwork feel.

### SUMMARY

In view of this, the present application provides a method for preparing a powder cosmetic with a gradient color effect and a powder cosmetic having a natural gradient blending effect.

In a first aspect, an embodiment of the present application provides a method for preparing a powder cosmetic with a gradient color effect, comprising the following steps:
Mixing: providing raw materials for preparing the powder cosmetic, and mixing them to obtain a mixed material system;
Color laydown: providing a mold, the mold having at least one cavity with an upward opening; taking at least one water-soluble pigment, diluting them separately to obtain at least one pigment solution, and dot-coating the at least one pigment solution onto at least one preset position on the bottom of the cavity;
Filling: after color laydown, filling a predetermined amount of the mixed material system into the mold, wherein the water-soluble pigment directionally diffuses within the mixed material system under the action of a concentration gradient;
Freezing: freezing the material system within the mold at a low temperature to obtain a powder masterbatch;
Demolding: separating the powder masterbatch from the mold;
Drying: drying the demolded powder masterbatch to obtain a powder cosmetic with a gradient color effect;
wherein the concentration of the pigment solution is 0.1 % to 10% by mass, preferably 0.5% to 5% by mass; and the viscosity of the mixed material system at 25°C is 1000 to 15000 cP, preferably 2000 to 5000 cP.

According to an embodiment of the first aspect of the present application, the mold is made of TPR, TPE, silicone, silicone rubber, rubber, or metal. The bottom of the cavity of the mold may or may not have a three-dimensional pattern design.

According to an embodiment of the first aspect of the present application, the gradient color effect is formed on the contact surface between the powder cosmetic and the bottom of the cavity, and extends into the interior of the powder cosmetic.

According to an embodiment of the first aspect of the present application, in the filling step, the mixed material system is filled into the mold from different directions to control the diffusion direction of the water-soluble pigment.

According to an embodiment of the first aspect of the present application, after the filling step, it further comprises an auxiliary diffusion step, the auxiliary diffusion step comprising: baking or microwave heating the mixed material system within the mold.

According to an embodiment of the first aspect of the present application, the baking temperature is 30 to 70°C, and the baking time is 5 minutes to 1 hour. Preferably, during the baking, the baking temperature increases in a gradient from the edge region to the central region of the mixed material system; more preferably, the baking temperature at the edge region of the mixed material system is 30 to 40°C, and the baking temperature at the central region of the mixed material system is 60 to 70°C.

According to an embodiment of the first aspect of the present application, the power of the microwave heating is 0.2 to 1 GHz, preferably 0.3 to 0.5 GHz; the microwave heating time is 20 seconds to 5 minutes, preferably 2 to 3 minutes.

According to an embodiment of the first aspect of the present application, the step of freezing the material system within the mold at a low temperature is performed in a liquid nitrogen quick-freezing device. Preferably, the temperature inside the liquid nitrogen quick-freezing device is -120°C to -70°C, preferably -95°C to -80°C.

According to an embodiment of the first aspect of the present application, the liquid nitrogen quick-freezing device is a liquid nitrogen tunnel, the material system passes through the liquid nitrogen tunnel on a conveyor belt within the liquid nitrogen tunnel at a speed of 0.6 to 1 m/min, and the time for the powder masterbatch passing through the liquid nitrogen tunnel is 6 to 10 minutes.

According to an embodiment of the first aspect of the present application, the drying comprises vacuum drying, freeze drying, microwave drying, supercritical fluid drying or any combination thereof; preferably, the drying is vacuum freeze drying.

According to an embodiment of the first aspect of the present application, the raw materials for preparing the powder cosmetic include a powder phase component, an oil phase component, and an aqueous phase component, the raw materials forming an oil-in-water emulsion system, wherein:
the powder phase component comprises one or more of a filler and a colorant;
the oil phase component comprises one or more of a moisturizing and emollient agent, an antioxidant, and a sunscreen agent;
the aqueous phase component comprises one or more of a solvent, a thickener, a film-forming agent, a moisturizing and emollient agent, an emulsifier, a preservative, and an active substance.

In a second aspect, an embodiment of the present application provides a powder cosmetic with a gradient color effect, prepared by the method according to any one of the preceding method; optionally, the powder cosmetic is selected from a powder cake, a blush, an eyeshadow, or a highlighter powder.

Compared with the prior art, the present application at least has the following beneficial effects:
in the method provided by the present application, water is used as a solvent, and a water-soluble pigment is dissolved therein to form a pigment solution with good fluidity. The pigment solution is dot-coated on the bottom of the mold cavity first, followed by material filling. Under the action of a concentration gradient, the pigment naturally diffuses from a high-concentration region to a low-concentration region, forming a softly transitioning gradient color effect. When two or more water-soluble pigments are used, pigments of different colors naturally blend during the diffusion process, forming multi-color blending and gradient color effects. By adjusting the initial concentration of the pigment solution and the viscosity of the mixed material system, the prepared powder cosmetic achieves a naturally graduated blending effect.

### BRIEF DESCRIPTION OF THE DRAWINGS

Other features, objectives, and advantages of the present application will become more apparent upon reading the detailed description of non-limiting embodiments with reference to the accompanying drawings, in which the same or similar reference numerals represent the same or similar features.
FIG. 1 is a schematic diagram of a product of Example 15 according to an embodiment of the present application;
FIG. 2 is a schematic diagram of a product of Example 16 according to an embodiment of the present application.

### DETAILED DESCRIPTION

To make the application purpose, technical solutions, and beneficial technical effects of the present application clearer, the present application is further described in detail below with reference to the embodiments. It should be understood that the embodiments described in this specification are only for explaining the present application and are not intended to limit the present application.

For the sake of brevity, only certain ranges are explicitly disclosed herein. However, it should be understood that any lower limit may be combined with any upper limit to recite a range not explicitly recited, as well as, any lower limit may be combined with any other lower limit to recite a range not explicitly recited, in the same way, any upper limit may be combined with any other upper limit to recite a range not explicitly recited. Additionally, although not explicitly recited, every point or individual value within a range is expressly included in the range. Thus, every point or individual value may serve as its own lower or upper limit and be combined with any other point or individual value or any other lower or upper limit, to form a range not explicitly recited.

In the description of the present application, it should be noted that, unless stated otherwise, "above", "below" include the base number, and "more" in "one or more" means two or more.

The above summary of the present application is not intended to describe every disclosed embodiment or every implementation of the present application. The description below more particularly exemplifies exemplary embodiments. Throughout the application, guidance is provided through a series of examples, which may be used in various combinations. In various instances, the listings are merely representative groups and should not be construed as exhaustive.

Existing multi-color and gradient powder cosmetics on the market are mostly pressed powders. Their manufacturing process is complex, production efficiency is low, and the surface cannot form a naturally graduated blending effect.

In view of the above problems, the present application provides a method for preparing a powder cosmetic with a gradient color effect and a powder cosmetic with a gradient color effect, which have a naturally graduated blending effect, enable mass production, and improve production efficiency.

### Preparation of Powder Cosmetic with Gradient Color Effect

In a first aspect, the present application provides a method for preparing a powder cosmetic with a gradient color effect, comprising the following steps:
Mixing: providing raw materials for preparing the powder cosmetic, and mixing them to obtain a mixed material system;
Color laydown: providing a mold, the mold having at least one cavity with an upward opening; taking at least one water-soluble pigment, diluting them separately to obtain at least one pigment solution, and dot-coating the at least one pigment solution onto at least one preset position on the bottom of the cavity;
Filling: after color laydown, filling a predetermined amount of the mixed material system into the mold, wherein the water-soluble pigment directionally diffuses within the mixed material system under the action of a concentration gradient;
Freezing: freezing the material system within the mold at a low temperature to obtain a powder masterbatch;
Demolding: separating the powder masterbatch from the mold;
Drying: drying the demolded powder masterbatch to obtain a powder cosmetic with a gradient color effect;
wherein the concentration of the pigment solution is 0.1 % to 10% by mass, preferably 0.5% to 5% by mass; and the viscosity of the mixed material system at 25°C is 1000 to 15000 cP, preferably 2000 to 5000 cP.

In the mixing step, the raw materials for preparing the powder cosmetic with a gradient color effect can be commercially available or self-made. It can be understood that the raw materials for preparing the powder cosmetic with a gradient color effect generally include a powder phase component, an oil phase component, and an aqueous phase component. The raw materials can be mixed by various methods commonly used in the prior art, adding the raw materials stepwise and mixing uniformly, such as mechanical stirring, and operations like homogenization and defoaming may be added during the mixing process. Different mixing methods and steps may be adopted depending on the raw materials, and the present application is not limited thereto.

In the preparation method provided in the embodiments of the present application, the pigment is dot-coated in the mold in advance, requiring the provision of a mold and the preparation of a water-soluble pigment solution. The mold needs to have at least one cavity with an upward opening for accommodating the dot-coated pigment and the mixed material system. When preparing the water-soluble pigment solution, one or more water-soluble pigments are taken, dissolved and diluted separately to obtain one or more pigment solutions, and the one or more pigment solutions are dot-coated onto at least one preset position on the bottom of the cavity.

When preparing the pigment solution according to the embodiments of the present application, the same pigment may be selected and diluted separately into solutions of multiple concentrations to form a concentration gradient; alternatively, two or more pigments may be selected and diluted separately into solutions of different concentrations or into solutions of multiple concentrations to form a concentration gradient; alternatively, two or more pigments may be mixed to obtain a pigment of a new color, which is then diluted and dot-coated. The use of various soluble pigments in the prior art is not limited and can be selected according to the requirements of the gradient color effect.

The embodiments of the present application do not limit the specific color of the water-soluble pigment. As an example, the water-soluble pigment may be one or more of commercially available water-soluble pigment products with the following color index numbers: Red 4 (CI 14700), Red 22 (CI 45380), Red 28 (CI 45410), Red 33 (CI 17200), Red 40 (CI 16035), Carmine (CI 75470), Blue 1 (CI 42090), Yellow 5 (CI 19140), Yellow 6 (CI 15985), Yellow 7 (CI 10316), Yellow 8 (CI 45350), Yellow 10 (CI 47005), Violet 2 (CI 60730), Orange 4 (CI 15510), Green 3 (CI 42053), Green 5 (CI 61570), Green 8 (CI 59040), etc.

The embodiments of the present application do not limit the specific sites and number of the preset positions, which can be selected according to design. As an example, the preset positions are evenly distributed around the outer ring of the cavity bottom, or evenly distributed on a certain side of the cavity bottom, or at a specific fixed point on the cavity bottom.

In the filling step, the predetermined amount can be set according to the volume of the powder cosmetic, the mold capacity, or the filling requirements, and the predetermined amount for each filling can be the same or different. After the mixed material system is filled into the colored mold in a predetermined amount, the water-soluble pigment will directionally diffuse within the mixed material system under the action of the concentration gradient.

In the embodiments of the present application, filling can be performed using a conventional filling machine or a pneumatic feeding device. A pneumatic feeding device refers to a device that fills the material system into the mold by pneumatic conveying. As an example, the pneumatic feeding device may include an air pump and a pipeline connected to the air pump, the air pump being used to provide the air pressure required for conveying, and the pipeline being used to fill the material into the mold. The present application is not limited thereto. When using a conventional filling machine, the material is filled under the action of gravity. If the fluidity of the material system meets the requirements, filling can be carried out at room temperature; when it is desired to increase the fluidity of the material system, the material system may be appropriately heated.

For powder cosmetics with a gradient color effect forming fine relief patterns, filling by means of a pneumatic feeding device is a more preferred method. The material system is filled into the mold by the pneumatic feeding device according to a preset amount, and the pressure assists the material system in filling into the sharp corners of the fine relief. Even if the viscosity of the material system is relatively high, it can still fill the sharp corners of the fine relief well and achieve good density, thereby solving the problems of porosity and looseness in the final product.

Specifically, the mixed material system can be pressed into the mold by air pressure. For example, the pneumatic feeding device includes a feeding pipe, an air pump, and a discharge pipe. The air pump provides conveying air pressure to draw the material from the feeding pipe into the pneumatic feeding device and then press it into the mold via the discharge pipe. Alternatively, the material system may be drawn into the mold by pneumatic pressure; for example, the pneumatic feeding device is connected to the mold and provides the conveying air pressure to draw the material into the mold. In some embodiments, the pressure in the pneumatic feeding device is 0.01 to 0.06 MPa, or 0.02 to 0.04 MPa.

After the filling step, a solvent removal step is also performed. Through process steps such as freeze-drying, the solvent is removed from the powder cosmetic. The solvent content in the final powder cosmetic generally needs to be controlled below 5%.

In the method provided by the present application, water is used as a solvent, and a water-soluble pigment is dissolved therein to form a pigment solution with good fluidity. The pigment solution is dot-coated on the bottom of the mold cavity first, followed by filling with the material system. Under the action of a concentration gradient, the pigment naturally diffuses from a high-concentration region to a low-concentration region, forming a gently transitioning gradient color effect. When two or more water-soluble pigments are used, pigments of different colors naturally blend during the diffusion process, forming multi-color blending and gradient effects.

The present application utilizes the diffusion of water-soluble pigment within the material system to achieve natural blending and gradient color effects. The initial concentration of the pigment solution and the viscosity of the mixed material system are key parameters. On one hand, the mixed material system needs to have an appropriate viscosity suitable for the diffusion of the water-soluble pigment. If the viscosity is too high, the pigment diffusion is hindered, and the gradient rendering effect is not obvious; if the viscosity is too low, the pigment may diffuse excessively within the material system, failing to achieve a natural gradient blending effect. On the other hand, the pigment solution needs to have an initial concentration suitable for diffusion within the mixed material system. If the initial concentration of the pigment solution is too low, the diffusion effect is weak, and the blending effect is insufficient; if the initial concentration of the pigment solution is too high, pigment accumulation may occur locally in the material system, resulting in an uneven surface after drying.

In the embodiments of the present application, by controlling the initial concentration of the pigment solution and the viscosity of the mixed material system within certain ranges, the prepared powder cosmetic achieves a natural gradient blending effect. The embodiments of the present application are suitable for preparing oil-in-water powder cosmetics.

As an example, the viscosity of the mixed material system at 25°C is 1000 cP, 2000 cP, 3000 cP, 4000 cP, 5000 cP, 6000 cP, 7000 cP, 8000 cP, 9000 cP, 10000 cP, 11000 cP, 12000 cP, 13000 cP, 14000 cP, 15000 cP, or in a range composed of any two of the above values.

As an example, the concentration of the pigment solution is 0.1%, 0.2%, 0.5%, 1%, 1.5%, 2%, 2.5%, 3%, 3.5%, 4%, 4.5%, 5%, 5.5%, 6%, 7%, 8%, 9%, 10% by mass, or in a range composed of any two of the above values.

The preparation method provided in the embodiments of the present application is a simple manufacturing process, avoiding the complexity of the traditional color mixing and pressing process, enabling mass production, and improving production efficiency.

In some embodiments, the mold is made of TPR, TPE, silicone, silicone rubber, rubber, or metal. The bottom of the cavity of the mold may or may not have a three-dimensional pattern.

The preparation method according to the embodiments of the present application can use molds made of the aforementioned TPR, TPE, silicone, silicone rubber, rubber, or metal, all of which can produce powder cosmetics with a naturally blended gradient color effect.

The bottom of the cavity of the selected mold can be planar, without a three-dimensional pattern; alternatively, the bottom of the cavity of the mold can have a three-dimensional pattern, which can be various. As an example, the three-dimensional pattern is a deer shape or an undulating wave shape, but the embodiments of the present application are not limited thereto.

In some embodiments, the gradient color effect is formed on the contact surface between the powder cosmetic and the bottom of the cavity, and extends into the interior of the powder cosmetic.

The pigment solution is dot-coated on the bottom of the cavity, so the gradient color effect is formed on the contact surface between the powder cosmetic and the bottom of the cavity. Under the action of the concentration gradient, the pigment solution diffuses in the thickness direction of the powder cosmetic, and the gradient color effect extends from the contact surface with the bottom of the cavity towards the direction away from the contact surface, i.e., towards the interior of the powder cosmetic.

In some embodiments, in the filling step, the mixed material system is filled into the mold from different directions to control the diffusion direction of the water-soluble pigment.

The filling of the mixed material system exerts an impact force that drives the diffusion of the water-soluble pigment, thereby controlling its diffusion direction. When the mixed material system is filled from different directions, the water-soluble pigment diffuses along different directions, which facilitates the formation of a natural gradient blending effect.

In the embodiments of the present application, during the filling of the mixed material system, it is filled into the mold from different directions. For example, the mixed material system is filled in different directions at a predetermined angle relative to the plane where the mold bottom is located. Preferably, the value of the predetermined angle is from 30° to 90°. As an example, the value of the predetermined angle is 30°, 40°, 45°, 50°, 60°, 70°, 80°, 90°, or in a range composed of any two of the above values.

For example, when the predetermined angle is 90°, the mixed material system is filled in a direction perpendicular to the plane of the mold bottom; when the predetermined angle is 60°, the mixed material system is filled in a direction inclined to the plane of the mold bottom.

In some embodiments, after the filling step, an auxiliary diffusion step is further included. The auxiliary diffusion step may include baking the mixed material system in the mold or microwave heating the mixed material system in the mold.

In the embodiments of the present application, after the filling step, an auxiliary diffusion step including baking or microwave heating is added. On one hand, part of the moisture in the mixed material system can be removed by baking or microwave heating; on the other hand, baking or microwave heating can also promote the formation of the gradient color effect. After filling is completed, the mixed material system is in a steady state. During the baking or microwave heating process, the temperature of the mixed material system rises, which helps the water-soluble pigment to continue diffusing in the steady-state mixed material system, making the gradient color effect more natural; at the same time, the water-soluble pigment is better adsorbed on the surface of the powder in the mixed material system, making the gradient color effect more stable. By adding the auxiliary diffusion step after the filling step, the embodiments of the present application can promote a more natural blending and gradient effect.

The embodiments of the present application do not limit the baking temperature and baking time, which can be set as needed. Preferably, the baking temperature is from 30 to 70°C, and the baking time is from 5 minutes to 1 hour.

A baking temperature within the range of 30 to 70°C ensures a relatively gentle baking process, allowing for slow moisture evaporation, which helps improve the uniformity of the powder cosmetic and also allows the water-soluble pigment to diffuse evenly in the steady-state mixed material system. As an example, the baking temperature may be 30°C, 35°C, 40°C, 45°C, 50°C, 55°C, 60°C, 65°C, 70°C, or in a range composed of any two of the above values.

A baking time within the range of 5 minutes to 1 hour ensures that part of the moisture in the mixed material system does not evaporate excessively on one hand, and makes the baking process relatively efficient, saving production time on the other hand. As an example, the baking time is 5 min, 10 min, 15 min, 20 min, 30 min, 40 min, 50 min, 1 h, or in a range composed of any two of the above values.

Furthermore, in the present application, during the directional diffusion of the water-soluble pigment in the mixed material system under the action of the concentration gradient, a phenomenon may occur where the diffusion speed of pigment molecules in the edge region of the material system is faster than that in the central region. This may be because the pigment molecules in the edge region of the material system have a lower probability of colliding with each other and diffuse faster, while the pigment molecules in the central region of the material system have a higher probability of colliding with each other and diffuse slower.

In this regard, in some embodiments, baking is used to assist diffusion, and the baking temperature is increased in a gradient from the edge region to the central region of the mixed material system. As an example, the baking temperature at the edge region of the mixed material system is from 30 to 40°C, and the baking temperature at the central region of the mixed material system is from 60 to 70°C, so that the auxiliary diffusion effect on the pigment molecules in the central region of the material system is greater than that on the pigment molecules in the edge region of the material system, reducing the difference in diffusion speed between the edge region and the central region when pigment molecules diffuse in the material system.

In other embodiments, microwave heating is used to assist diffusion. As an example, the power of the microwave heating is from 0.2 to 1 GHz, preferably from 0.3 to 0.5 GHz, and the microwave heating time is from 20 seconds to 5 minutes, preferably from 2 to 3 minutes. As an example, the power of the microwave heating may be 0.2 GHz, 0.3 GHz, 0.4 GHz, 0.5 GHz, 0.6 GHz, 0.7 GHz, 0.8 GHz, 0.9 GHz, 1.0 GHz, or in a range composed of any two of the above values. As an example, the microwave heating time may be 20 s, 30 s, 40 s, 50 s, 1 min, 1.2 min, 1.4 min, 1.5 min, 1.6 min, 1.8 min, 2 min, 2.5 min, 3 min, 3.5 min, 4 min, 4.5 min, 5 min, or in a range composed of any two of the above values.

Microwave heating exhibits a center effect, meaning that microwaves reflect in the cavity, the central area is located in the overlapping region of the microwave field, where the microwave energy density is relatively high, and the microwave energy decreases towards the edge region. Therefore, through relatively low-power microwave heating in the present application, under the premise that the mixed material system does not dry immediately, the central region is first subjected to microwave radiation, triggering accelerated molecular motion, and the auxiliary effect on diffusion is higher than that on the edge region. Due to the above characteristics of microwave heating, auxiliary diffusion can be achieved in a relatively short time, solving the situation where the diffusion speed of pigment molecules in the edge region is greater than that in the central region when pigment molecules diffuse in the material system. This minimizes the interference factors of the gradient color effect, resulting in a more natural and softer gradient.

In some embodiments, the step of freezing the material system in the mold at a low temperature is performed in a liquid nitrogen quick-freezing device. Preferably, the temperature inside the liquid nitrogen quick-freezing device is from -120°C to -70°C, preferably from - 95°C to -80°C.

As an example, the liquid nitrogen quick-freezing device may be a liquid nitrogen tunnel, a liquid nitrogen freezer, an ultra-low temperature refrigerator, etc. These devices are commercially available, and the present application is not limited thereto.

The raw material components in the mixed material system undergo molecular quick-freezing in an extremely low-temperature environment in the liquid nitrogen quick-freezing device. The mixed material system contains components such as oils and emulsions. During the freezing and setting process, the mixed material system passes through a maximum ice crystal formation zone, which is approximately in the temperature range of -10 to 0°C, where about 80% of the liquid (water, emulsions, etc.) turns into ice. Unlike the ordinary freezing performed before demolding in the prior art, during the molecular quick-freezing process completed in the liquid nitrogen tunnel in the present application, the powder masterbatch quickly passes through the maximum ice crystal formation zone. The distribution of ice crystals in the powder masterbatch is finer, more uniform, and denser. During the subsequent drying process, the removal of fine and uniform ice crystals does not affect the formed gradient color effect, ensuring a natural, soft and continuous gradient, and resulting in a product with a softer skin feel after freeze-drying, smaller and more controllable dimensional changes, a more stable product structure, and better drop resistance.

In some embodiments, the liquid nitrogen quick-freezing device is a liquid nitrogen tunnel. The material system passes through the liquid nitrogen tunnel on a conveyor belt within the liquid nitrogen tunnel at a speed of 0.6 to 1 m/min, and the time for the powder masterbatch passing through the liquid nitrogen tunnel is from 6 minutes to 10 minutes.

A liquid nitrogen tunnel, also known as a liquid nitrogen tunnel freezer, is a device that uses liquid nitrogen as a cooling source, adopts a continuous production mode, and uses a conveyor belt to feed materials into the freezing tunnel, enabling continuous input and output and rapid freezing of materials.

Through research on the freezing curve of the mixed material system of the powder cosmetic and the energy transfer characteristics in the liquid nitrogen quick-freezing device, the present application also proposes optimal liquid nitrogen quick-freezing condition parameters suitable for the preparation of the powder cosmetic of the present application. Performing liquid nitrogen quick-freezing of the powder masterbatch under the above condition parameters can make the finally prepared powder cosmetic have a uniform and dense texture, the best skin feel, and the stability and drop resistance of the product structure reach the optimum. The powder masterbatch passes through the liquid nitrogen tunnel on the conveyor belt within the liquid nitrogen tunnel at a speed of 0.6 to 1 m/min, and the time for the powder masterbatch passing through the liquid nitrogen tunnel is from 6 minutes to 10 minutes.

In some embodiments, the drying comprises one or more of vacuum drying, freeze drying, microwave drying, and supercritical fluid drying. Preferably, the drying is vacuum freeze drying.

In the embodiments of the present application, the demolded powder masterbatch is dried by vacuum freeze-drying technology. Freeze-drying involves freezing the water-containing material below the freezing point to convert water into ice, and then, in a vacuum environment or under relatively high vacuum conditions, sublimating the finely and uniformly distributed ice crystals in the powder masterbatch into water vapor for removal. Such process maximizes the retention of the ingredients, color, and fragrance of the raw materials. The pigment is naturally adsorbed on the powder surface and does not migrate with the liquid water, thus ensuring the stable maintenance of the rendering effect throughout the drying process.

The embodiments of the present application may also use methods such as vacuum drying, freeze drying, microwave drying, and supercritical fluid drying for drying, or any combination of the above multiple drying methods.

In some embodiments, the raw materials for preparing the powder cosmetic include a powder phase component, an oil phase component, and an aqueous phase component, the raw materials forming an oil-in-water emulsion system, wherein:
the powder phase component comprises one or more of a filler and a colorant;
the oil phase component comprises one or more of a moisturizing and emollient agent, an antioxidant, and a sunscreen agent;
the aqueous phase component comprises one or more of a solvent, a thickener, a film-forming agent, a moisturizing and emollient agent, an emulsifier, a preservative, and an active substance.

In the embodiments of the present application, the raw materials form an oil-in-water emulsion system. The oil-in-water emulsion system has water as the continuous phase, which allows the water-soluble pigment to flow better, resulting in a more natural gradient effect.

In some embodiments, the thickener and the film-forming agent are selected from natural water-soluble polymers or derivatives thereof, synthetic water-soluble polymers, or water-based inorganic thickeners.

Natural water-soluble polymers or derivatives thereof are selected from at least one of alginic acid, agar, carrageenan, Xanthan, gellan gum, chondrus crispus, xanthan gum, guar gum, tamarind gum, tara gum, gum arabic, tragacanth gum, Peruvian carob, pectin, arabinogalactan, wheat protein, soy protein, gelatin, casein, chitosan, curdlan polysaccharide, cyclodextrin, hyaluronic acid, konjac glucomannan, tremella polysaccharide, curdlan, microcrystalline cellulose, methyl cellulose, hydroxyethyl cellulose, hydroxypropyl cellulose, starch phosphate, starch, sodium starch hydroxypropylate, sodium polyacrylate grafted starch, hydroxypropyl guar gum.

Synthetic water-soluble polymers are selected from at least one of acrylates/C10-30 alkyl acrylate crosspolymer, carbomer, acrylates/octylacrylamide copolymer, acrylates/ethylhexyl acrylate copolymer, sodium acrylate/sodium acryloyldimethyl taurate copolymer, acrylates/octylacrylamide copolymer, styrene/acrylates copolymer, acrylates copolymer, hydroxyethyl acrylate/sodium acryloyldimethyl taurate copolymer, polyacrylate crosspolymer-6, acrylates/steareth-20 methacrylate copolymer, ammonium acryloyldimethyltaurate/beheneth-25 methacrylate crosspolymer, ammonium acrylates copolymer, sodium polyacrylate, sodium polyacryloyldimethyl taurate, polyacrylamide, polyvinylpyrrolidone, polyvinyl alcohol, VP/VA copolymer, polyurethane-1, polyurethane-35.

Water-based inorganic thickeners are selected from at least one of magnesium aluminum silicate, lithium magnesium sodium silicate, magnesium lithium silicate, sodium magnesium silicate, montmorillonite or derivatives thereof.

In some embodiments, the moisturizing and emollient agent is selected from at least one of alcohols, silicone oils, mineral oils, synthetic oils, animal and vegetable oils or derivatives thereof.

Animal and vegetable oils or derivatives thereof are selected from at least one of glycerin, propylene glycol, dipropylene glycol, pentylene glycol, butylene glycol, ethylene glycol, hexylene glycol, sorbitol, xylitol, polyethylene glycol, dimethicone, caprylyl methicone, phenyl trimethicone, cetyl dimethicone, C30-45 alkyl dimethicone/polypropylsilsesquioxane crosspolymer, bis-PEG-18 methyl ether dimethyl silane, white mineral oil, petrolatum, hydrogenated polyisobutene, lanolin and derivatives thereof, octyldodecanol, caprylic/capric triglyceride, ethylhexyl palmitate, bis-diglyceryl polyacyladipate-2, isononyl isononanoate, jojoba seed oil, coconut oil, hydrogenated coconut glycerides, meadowfoam seed oil, olive oil, squalane, C9-12 alkane, coco-caprylate/caprate, tocopheryl acetate, or methyl gluceth-10.

In some embodiments, the filler is selected from at least one of mica, talc, synthetic fluorphlogopite, kaolin, bentonite, boron nitride, bismuth oxychloride, silica, potassium sodium aluminum silicate, lauroyl lysine, magnesium stearate, magnesium myristate, calcium aluminum borosilicate, tin oxide, sodium calcium borosilicate, aluminum hydroxide, zinc stearate, starch and derivatives thereof, or plastic microspheres.

In some embodiments, the preservative is selected from at least one of phenoxyethanol, parabens, chlorphenesin, potassium sorbate, sodium benzoate, or preservative enhancers.

Preservative enhancers are selected from at least one of caprylyl glycol, hydroxyacetophenone, ethylhexylglycerin, 1,2-hexanediol, caprylhydroxamic acid, 1,2-pentanediol, glyceryl caprylate, or p-cymene.

In a second aspect, an embodiment of the present application provides a powder cosmetic with a gradient color effect, prepared by any one of the preceding methods. Optionally, the powder cosmetic is selected from a powder cake, a blush, or an eyeshadow.

The powder cosmetic prepared in the embodiments of the present application has a natural gradient blending effect.

In some embodiments, the powder cosmetic with a gradient color effect is selected from a powder cake, a blush, an eyeshadow, or a highlighter powder. The powder cosmetic with a gradient color effect may also include products with other functions.

### Examples

The following examples describe the disclosure of the present application in more detail. These examples are provided for illustrative purposes only, as various modifications and variations within the scope of the disclosure of the present application will be apparent to those skilled in the art. Unless otherwise stated, all parts, percentages, and ratios reported in the following examples are based on mass, and all reagents used in the examples are commercially available or synthesized according to conventional methods, and can be used directly without further processing. Furthermore, the instruments used in the examples are commercially available.

### Example 1

(1) Mixing: The following raw materials were provided: Phase A: water, a binder, potassium chloride solution, an emulsifier, oil, VE; Phase B: synthetic fluorphlogopite, silica; Phase C: pearlescent agent; Phase D: preservative. The temperature during the mixing process was controlled within the range of 60-65°C. Phase A was heated, and Phase B, Phase C, and Phase D were added, followed by emulsification and homogenization. The material after emulsification and homogenization was vacuum defoamed to obtain a mixed material system. The viscosity of the mixed material system at 25°C was 5000 cP, and it was set aside for filling.
(2) Color laydown: A silicone mold was provided. The silicone mold had a cavity with an upward opening, and the bottom of the cavity had a relief three-dimensional pattern. The water-soluble pigment CI 42090 was diluted with water to prepare a pigment solution with a concentration of 3% by mass. The pigment solution was spotted onto preset positions on the bottom of the cavity of the silicone mold.
(3) Filling: A predetermined amount of the mixed material system was filled into the color-distributed mold from the center position of the mold.
(4) Freezing: The mold filled with the mixed material system was placed in a freezer at -15°C for freezing to obtain a powder masterbatch.
(5) Demolding: The powder masterbatch was separated from the mold.
(6) Drying: The demolded powder masterbatch was baked to obtain a powder cosmetic.

### Examples 2-5

Examples 2-5 differ from Example 1 in that the concentration (mass percentage) of the pigment solution or the viscosity of the mixed material system was different.

### Examples 6-9

Examples 6-9 differ from Example 1 in that an auxiliary diffusion step was performed on the material system within the mold after the filling step.

The auxiliary diffusion step in Example 6 was baking, involving uniform baking of the mixed material system at 50°C.

The auxiliary diffusion step in Example 7 was baking, with the baking temperature increasing in a gradient from the edge region to the central region of the mixed material system. The baking temperature at the edge region of the mixed material system was 30°C, and the baking temperature at the central region of the mixed material system was 70°C.

The auxiliary diffusion steps in Examples 8 and 9 were microwave heating. The microwave frequencies within the microwave cavity were 0.3 GHz and 0.5 GHz, and the times for microwave heating were 3 min and 2 min, respectively.

### Examples 10-12

Examples 10-12 differ from Example 1 in that the step of freezing the material system within the mold was carried out in a liquid nitrogen tunnel.

### Example 13

Example 13 differs from Example 1 in that the drying step employed vacuum freeze-drying. Specifically, the vacuum freeze-drying comprised pre-freezing, sublimation drying, and desorption drying. The pre-freezing specifically comprised freezing at -40°C to -50°C for 0.5 h to 2 h. The sublimation drying comprised: first performing a temperature increase treatment to a temperature ranging from -3°C to 3°C, wherein the temperature increase treatment comprised a plurality of first temperature increase stages and a plurality of first holding stages, and the first temperature increase stages and the first holding stages were carried out alternately; each of the first temperature increase stages involved a temperature increase of 0.5 to 1°C/min for 10 to 20 minutes, and the time for the first holding was 1.5 to 2 hours. The desorption drying comprised: performing a drying treatment at a temperature of 20°C to 60°C, wherein the drying treatment comprised a plurality of second temperature increase stages and a plurality of second holding stages, and the second temperature increase stages and the second holding stages were carried out alternately; each of the second temperature increase stages involved a temperature increase of 0.5 to 1°C/min for 10 to 20 minutes, and the time for the second holding was 1.5 to 2 hours.

### Example 14

Example 14 differs from Example 1 in that microwave-assisted diffusion was performed on the material system within the mold after the filling step; the step of freezing the material system within the mold was carried out in a liquid nitrogen tunnel; the drying step employed vacuum freeze-drying, and the specific steps of freeze-drying were the same as those in Example 13.

### Example 15

Example 15 differs from Example 1 in that baking was performed on the material system within the mold after the filling step; the step of freezing the material system within the mold was carried out in a liquid nitrogen tunnel; the drying step employed vacuum freeze-drying, and the specific steps of freeze-drying were the same as those in Example 13; in step (2) of the color laydown, the water-soluble pigments CI 19140 and CI 42090 were pre-mixed to obtain a combined pigment of the desired color, then diluted with water in different ratios to obtain several pigment solutions of different concentrations, and these pigment solutions were dot-coated onto preset positions on the outer periphery of the bottom of the mold cavity.

### Example 16

Example 16 differs from Example 15 in step (2) of the color laydown. The water-soluble pigment CI 42090 was diluted with water in a certain ratio (blue); the water-soluble pigment CI 17200 was diluted with water in a certain ratio (red); the water-soluble pigments CI 42090 and CI 17200 were mixed in a certain ratio and then diluted with water (purple). The blue and red pigment solutions were dot-coated onto diagonal positions on the bottom of the mold, respectively, while the purple pigment solution was dot-coated onto the other two diagonal positions.

### Comparative Examples 1-6

Comparative Example 1 differs from Example 1 in that, before filling, a syringe was used to inject the pigment solution into the interior of the mixed material system, which was then filled.

Comparative Example 2 differs from Example 1 in that, immediately after filling, a syringe was used to inject the pigment solution into the bottom of the mold cavity.

Comparative Examples 3-6 differ from Example 1 in that the concentration of the pigment solution and the viscosity of the mixed material system were different.

The parameters for mixing, color laydown, filling, baking, freezing/demolding, and drying for Examples 1-16 and Comparative Examples 1-6 were shown in Table 1.

**Table 1: Process Parameters of Examples 1-16 and Comparative Examples 1-6**

| | Mixing | Color laydown | Filling and auxiliary diffusion | | | Freezing/ demolding | Drying |
|---|---|---|---|---|---|---|---|
| | Viscosity of the mixed material system (25°C) | Concentration of pigment solution wt% | Auxiliary diffusion | Baking parameters | Microwave parameters | Freezing temperature | Drying method |
| Example 1 | 5000cP | 3% | No | / | / | -15°C | Baking drying |
| Example 2 | 2000cP | 0.5% | No | / | / | -15°C | Microwave drying |
| Example 3 | 1000cP | 0.1% | No | / | / | -15°C | Microwave drying |
| Example 4 | 10000cP | 5% | No | / | / | -15°C | Baking drying |
| Example 5 | 15000cP | 10% | No | / | / | -15°C | Microwave drying |
| Example 6 | 5000cP | 3% | Baking | 50°C, 30min | / | -15°C | Baking drying |
| Example 7 | 5000cP | 3% | Gradient baking | 30~70°C , 20min | / | -15°C | Baking drying |
| Example 8 | 5000cP | 3% | Microwave | / | 0.3GHz, 3min | -15°C | Baking drying |
| Example 9 | 5000cP | 3% | Microwave | / | 0.5GHz, 2min | -15°C | Baking drying |
| Example 10 | 5000cP | 3% | No | / | / | -70°C | Baking drying |
| Example 11 | 5000cP | 3% | No | / | / | -80°C | Baking drying |
| Example 12 | 5000cP | 3% | No | / | / | -95°C | Baking drying |
| Example 13 | 5000cP | 3% | No | / | / | -15°C | Freeze-drying |
| Example 14 | 5000cP | 3% | Microwave | / | 0.3GHz, 3min | -80°C | Freeze-drying |
| Example 15 | See Example 15 for details | | Baking | 50°C, 30min | / | -80°C | Freeze-drying |
| Example 16 | See Example 16 for details | | Baking | 50°C, 30min | / | -80°C | Freeze-drying |
| Comparative Example 1 | See Comparative Example 1 for details | | | | | | |
| Comparative Example 2 | See Comparative Example 2 for details | | | | | | |
| Comparative Example 3 | 500cP | 3% | No | / | / | -15°C | Baking drying |
| Comparative Example 4 | 20000cP | 3% | No | / | / | -15°C | Baking drying |
| Comparative Example 5 | 5000cP | 0.05% | No | / | / | -15°C | Baking drying |
| Comparative Example 6 | 5000cP | 20% | No | / | / | -15°C | Baking drying |

### Test Methods

Powder cakes were prepared according to the methods of Examples 1-16 and Comparative Examples 1-6, with 100 powder cakes prepared for each example or comparative example. Appearance observation and drop tests were conducted.

### 1) Appearance Observation

The appearance of the powder cakes was observed with the naked eye or with the aid of a microscope, including observation of the gradient color effect, with a focus on whether the gradient color effect was natural and uniform, and the flatness of the gradient color surface.

The recorded appearance was based on the observation results of more than 90% of the 100 powder cakes.

### 2) Drop Test

The rotating screws of the drop test stand were adjusted to set the flat plate at a height of 30 cm. The powder cake was placed in the center position of the test height, with its front side facing upward.

The first drop test was performed as followed: the button of the test stand was pressed to drop the powder block onto the rigid plate. The powder block that had fallen onto the table surface was picked up and inspected for any cracks or breakage on its surface. If the powder block cracked or broke, it was excluded from further drop testing. If the powder block showed no abnormality, the second drop test was conducted. The operational steps for the second drop test were the same as those for the first drop test. If the powder block cracked or broke, it was excluded from further drop testing. If the powder block showed no abnormality, the third drop test was conducted. The operational steps for the third drop test were the same as those for the first drop test.

The ratio of the number of broken powder cakes in each drop test to the total number of powder cakes subjected to that drop test was the breakage rate for that drop test. The first, second, and third breakage rates for Examples 1-16 and Comparative Examples 1-6 were recorded separately. The scoring criteria for drop resistance are shown in Table 2.

**Table 2 Scoring Criteria for Drop Resistance**

| Sum of breakage rates for 3 drops | Less than 5% | 5%~10% | 10%~15% | 15%~20% | 20%~25% | 25%~30% |
|---|---|---|---|---|---|---|
| Drop resistance score | 5 points | 4 points | 3 points | 2 points | 1 point | 0 point |

The above test results for Examples 1-16 and Comparative Examples 1-6 were recorded in Table 3.

**Table 3 Test Results for Examples 1-16 and Comparative Examples 1-6**

| | **Appearance Observation of Gradient Color Effect** | | Drop Resistance Score |
|---|---|---|---|
| | Gradient Color Effect | Flatness of Surface with Gradient Color | |
| Example 1 | A gradient color effect can be formed. During preparation, it was observed that the pigment diffusion rate at the edge region of the material system was faster than that at the central region, which affected the gradient color effect of some patterns. | Flat | 3 |
| Example 2 | A gradient color effect can be formed. During preparation, it was observed that the pigment diffusion rate at the edge region of the material system was faster than that at the central region, which affected the gradient color effect of some patterns. | Flat | 3 |
| Example 3 | A gradient color effect can be formed. During preparation, it was observed that the pigment diffusion rate at the edge region of the material system was faster than that at the central region, which affected the gradient color effect of some patterns. | Flat | 2 |
| Example 4 | A gradient color effect can be formed. During preparation, it was observed that the pigment diffusion rate at the edge region of the material system was faster than that at the central region, which affected the gradient color effect of some patterns. | Flat | 2 |
| Example 5 | A gradient color effect can be formed. During preparation, it was observed that the pigment diffusion rate at the edge region of the material system was faster than that at the central region, which affected the gradient color effect of some patterns. | Flat | 2 |
| Example 6 | A gradient color effect can be formed, with a larger gradient range compared to Examples 1-5. During preparation, it was observed that the pigment diffusion rate at the edge region of the material system was faster than that at the central region, which affected the gradient color effect of some patterns. | Flat | 3 |
| Example 7 | A gradient color effect can be formed, with a larger gradient range compared to Examples 1-5. During preparation, the phenomenon where the pigment diffusion rate at the edge region of the material system was faster than that at the central region did not occur, resulting in a softer and more natural gradient transition. | Flat | 3 |
| Example 8 | A gradient color effect can be formed, with a larger gradient range compared to Examples 1-5. During preparation, the phenomenon where the pigment diffusion rate at the edge region of the material system was faster than that at the central region did not occur, resulting in a softer and more natural gradient transition. | Flat | 3 |
| Example 9 | A gradient color effect can be formed, with a larger gradient range compared to Examples 1-5. During preparation, the phenomenon where the pigment diffusion rate at the edge region of the material system was faster than that at the central region did not occur, resulting in a softer and more natural gradient transition. | Flat | 3 |
| Example 10 | A gradient color effect can be formed, with more vivid colors compared to Examples 1-5. During preparation, it was observed that the pigment diffusion rate at the edge region of the material system was faster than that at the central region, which affected the gradient color effect of some patterns. | Flat | 4 |
| Example 11 | A gradient color effect can be formed, with more vivid colors compared to Examples 1-5. During preparation, it was observed that the pigment diffusion rate at the edge region of the material system was faster than that at the central region, which affected the gradient color effect of some patterns. | Flat | 4 |
| Example 12 | A gradient color effect can be formed, with more vivid colors compared to Examples 1-5. During preparation, it was observed that the pigment diffusion rate at the edge region of the material system was faster than that at the central region, which affected the gradient color effect of some patterns. | Flat | 4 |
| Example 13 | A gradient color effect can be formed, with more vivid colors compared to Examples 1-5. During preparation, it was observed that the pigment diffusion rate at the edge region of the material system was faster than that at the central region, which affected the gradient color effect of some patterns. | Flat | 4 |
| Example 14 | The best gradient color effect was achieved, with a soft and natural gradient transition. During preparation, the phenomenon where the pigment diffusion rate at the edge region of the material system was faster than that at the central region did not occur. The colors were vivid, and no color loss occurred after drying. | Flat | 5 |
| Example 15 | A good gradient color effect was achieved, with a soft and natural gradient transition, vivid colors, and no color loss after drying. | Flat | 5 |
| Example 16 | A good gradient color effect was achieved, with a soft and natural gradient transition, vivid colors, and no color loss after drying. | Flat | 5 |
| Comparative Example 1 | A directional, natural gradient effect cannot be formed. | Flat | 1 |
| Comparative Example 2 | Localized pigment diffusion, with a too small diffusion range. | Uneven | 0 |
| Comparative Example 3 | Excessive pigment diffusion in the material system, resulting in no gradient transition. | Flat | 0 |
| Comparative Example 4 | Pigment diffusion was hindered, and no gradient effect was observable. | Flat | 1 |
| Comparative Example 5 | Color and gradient effect were not obvious. | Flat | 1 |
| Comparative Example 6 | Localized pigment accumulation occurred. | Uneven | 0 |

From the test results shown in Table 3, it can be seen that Examples 1-16, employing the method for preparing powder cosmetics with a gradient color effect provided in the present application, underwent color laydown, material filling into the mold, followed by freezing, drying, and demolding to obtain block-shaped powder cosmetics. Compared with Comparative Examples 1-6, which did not use the preparation method of the present application, Comparative Examples 1-6 failed to form a gradient color effect, or the color and gradient effect were not obvious. Examples 1-16 showed significant improvements in filling efficiency, naturalness and uniformity of the gradient color effect, and flatness of surface with gradient color, and also played a significant role in reducing the breakage rate of the powder cakes in drop tests.

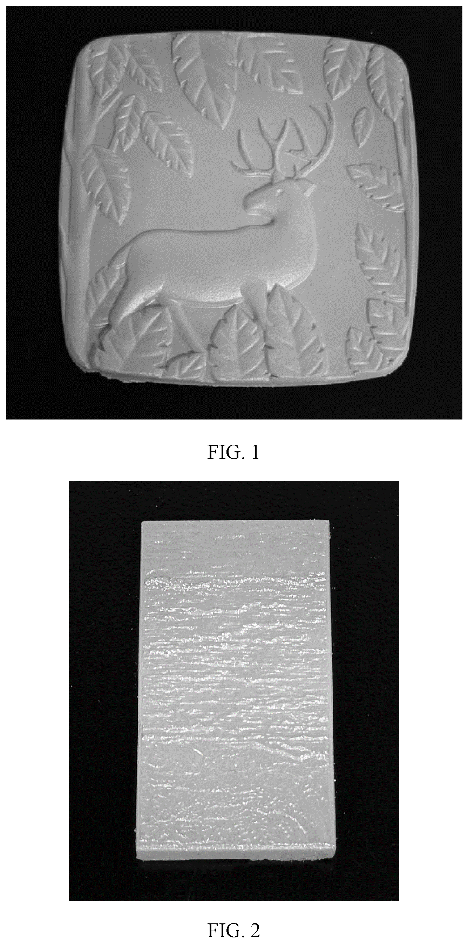

In the method for preparing powder cosmetics with a gradient color effect provided in the present application, parameters such as the initial concentration of the pigment solution, the viscosity of the mixed material system, and the temperature and time control for solidifying the powder masterbatch at low temperatures also influence the achievement of the technical effects of the present application. The present application also provides optional preferred ranges for these process parameters to further enhance various properties of the powder cosmetics with gradient color effect prepared according to the method provided herein. For example, in Examples 6-9, auxiliary diffusion after filling resulted in a larger gradient range compared to Examples 1-5. Particularly, auxiliary diffusion via gradient baking and microwave heating avoided the phenomenon during preparation where the pigment molecular diffusion rate at the edge region of the material system was faster than that at the central region, reducing interference with the gradient effect and achieving a softer, more natural gradient transition. In Examples 10-12, ultra-low temperature pre-freezing in a liquid nitrogen tunnel not only allowed the formation of a gradient color effect but also resulted in more vivid colors compared to Examples 1-5, with no color loss after drying. Example 13, employing vacuum freeze-drying including pre-freezing, sublimation drying, and desorption drying, also allowed the formation of a gradient color effect with more vivid colors compared to Examples 1-5 and no color loss after drying. Examples 14-16 achieved a good gradient color effect by adjusting process parameters, with reduced interference to the gradient effect, a soft and natural gradient transition, vivid colors, and no color loss after drying. Please refer to FIG. 1 for the product effect of Example 15 and FIG. 2 for the product effect of Example 16.

Comparative Examples 1-6, which did not implement the preparation steps using the method provided in the present application, failed to obtain powder cosmetics with a gradient color effect.

In Comparative Example 1, the pigment solution was injected into the mixed material system using a syringe before filling, and then the material was filled. While a shading effect was achieved on the surface, a directional, natural gradient shading effect could not be formed. In Comparative Example 2, the pigment solution was injected into the bottom of the mold cavity immediately after filling. The pigment could only diffuse locally, and air was introduced into the material system, compromising its internal density. The resulting powder product exhibited holes, cracks, and pigment spots, and showed poor drop resistance. In Comparative Example 3, the viscosity of the material system was too low, leading to excessive pigment diffusion. If multiple colors were used, they might have mixed completely into a single color. Moreover, the low viscosity resulted in a powder cake that was too soft after forming and exhibited poor drop resistance. In Comparative Example 4, the viscosity of the material system was too high, hindering pigment diffusion and resulting in an indistinct blending effect. In Comparative Example 5, the concentration of the pigment solution was too low, making the shading effect indistinct. In Comparative Example 6, the concentration of the pigment solution was too high, leading to localized pigment accumulation in the material system and an uneven surface after drying.

The above Examples 1-16 and Comparative Examples 1-6 fully show that the preparation method provided in the present application has outstanding beneficial effects and can be used to prepare powder cosmetics with natural shading and gradient color effects.

Described above are merely specific implementations of the present application, but the protection scope of the present application is not limited thereto. Any skilled person who is familiar with this art would readily conceive of various equivalent modifications or substitutions within the technical scope of the disclosure of the present application, and these modifications or substitutions shall fall within the protection scope of the present application. Therefore, the protection scope of the present application shall be subject to the protection scope of the claims.

## Claims

1. A method for preparing a powder cosmetic with a gradient color effect, comprising the following steps:
mixing: providing raw materials for preparing the powder cosmetic, and mixing them to obtain a mixed material system;
color laydown: providing a mold, the mold having at least one cavity with an upward opening; taking at least one water-soluble pigment, diluting them separately to obtain at least one pigment solution, and dot-coating the at least one pigment solution onto at least one preset position on a bottom of the cavity;
filling: after color laydown, filling a predetermined amount of the mixed material system into the mold, wherein the water-soluble pigment directionally diffuses within the mixed material system under the action of a concentration gradient;
freezing: freezing the material system within the mold at a low temperature to obtain a powder masterbatch;
demolding: separating the powder masterbatch from the mold;
drying: drying the demolded powder masterbatch to obtain a powder cosmetic with a gradient color effect;
wherein a concentration of the pigment solution is from 0.1 % to 10% by mass, preferably from 0.5% to 5% by mass; and a viscosity of the mixed material system at 25°C is 1000 to 15000 cP, preferably 2000 to 5000 cP.

2. The method for preparing a powder cosmetic with a gradient color effect according to claim 1, wherein the mold is made of TPR, TPE, silicone, silicone rubber, rubber, or metal; and the bottom of the cavity of the mold may or may not have a three-dimensional pattern design.

3. The method for preparing a powder cosmetic with a gradient color effect according to claim 1, wherein the gradient color effect is formed on a contact surface between the powder cosmetic and the bottom of the cavity, and extends into the interior of the powder cosmetic.

4. The method for preparing a powder cosmetic with a gradient color effect according to claim 1, wherein in the filling step, the mixed material system is filled into the mold from different directions to control the diffusion direction of the water-soluble pigment.

5. The method for preparing a powder cosmetic with a gradient color effect according to claim 1, wherein after the filling step, it further comprises an auxiliary diffusion step, the auxiliary diffusion step comprising: baking or microwave heating the mixed material system within the mold.

6. The method for preparing a powder cosmetic with a gradient color effect according to claim 5, wherein a baking temperature is from 30 to 70°C, and a baking time is from 5 minutes to 1 hour; preferably, during the baking, the baking temperature increases in a gradient from the edge region to the central region of the mixed material system; more preferably, the baking temperature at the edge region of the mixed material system is from 30 to 40°C, and the baking temperature at the central region of the mixed material system is from 60 to 70°C.

7. The method for preparing a powder cosmetic with a gradient color effect according to claim 5, wherein the microwave heating has a power of from 0.2 to 1 GHz, preferably from 0.3 to 0.5 GHz; a microwave heating time is from 20 seconds to 5 minutes, preferably from 2 to 3 minutes.

8. The method for preparing a powder cosmetic with a gradient color effect according to claim 1, wherein the step of freezing the material system within the mold at a low temperature is performed in a liquid nitrogen quick-freezing device; preferably, the temperature inside the liquid nitrogen quick-freezing device is from -120°C to -70°C, preferably from -95°C to -80°C.

9. The method for preparing a powder cosmetic with a gradient color effect according to claim 8, wherein the liquid nitrogen quick-freezing device is a liquid nitrogen tunnel, the material system passes through the liquid nitrogen tunnel on a conveyor belt within the liquid nitrogen tunnel at a speed of 0.6 to 1 m/min, and a time for the powder masterbatch passing through the liquid nitrogen tunnel is from 6 minutes to 10 minutes.

10. The method for preparing a powder cosmetic with a gradient color effect according to claim 1, wherein the drying comprises vacuum drying, freeze drying, microwave drying, supercritical fluid drying or any combination thereof; preferably, the drying is vacuum freeze drying.

11. The method for preparing a powder cosmetic with a gradient color effect according to claim 1,wherein the raw materials for preparing the powder cosmetic include a powder phase component, an oil phase component, and an aqueous phase component, the raw materials forming an oil-in-water emulsion system, wherein:
the powder phase component comprises one or more of a filler and a colorant;
the oil phase component comprises one or more of a moisturizing and emollient agent, an antioxidant, and a sunscreen agent;
the aqueous phase component comprises one or more of a solvent, a thickener, a film-forming agent, a moisturizing and emollient agent, an emulsifier, a preservative, and an active substance.

12. A powder cosmetic with a gradient color effect prepared by the method according to any one of claims 1 to 11; optionally, the powder cosmetic is selected from a powder cake, a blush, an eyeshadow, or a highlighter powder.
